# EUROPEAN PATENT APPLICATION

(11) **EP 3 430 971 A1**
(43) Date of publication of application: **23.01.2019**
(21) Application number: 17200024.2
(22) Date of filing: 03.11.2017
(51) Int. Cl.: A61B 1/267, A61B 1/24

(54) **MEDICAL DEVICE COMPRISING A TOOTH PROTECTIVE DEVICE FOR ORAL INTUBATION PROCEDURES**

(30) Priority: 19.07.2017 EP 17182108
(71) Applicant: Universität Zürich, 8006 Zürich (CH)
(72) Inventor: SCHLICKER, Andreas, 79268 Bötzingen (CH); SCHRÖDER, Carsten, 8003 Zürich (CH); WEIDNER, Sascha, 8057 Zürich (CH)
(74) Representative: Schulz Junghans Patentanwälte PartGmbB

(57) **Abstract**

The invention relates to a medical device (1) for oral procedures, particularly for use in oral intubation and fixation procedures, comprising at least one protective device (2) for teeth, the protective device (2) comprising the a rigid support component (3), and at least one push button (4), arranged at the support component (3), wherein each of the at least one the push button (4) comprises a rigid surface (42) that is facing towards the teeth, when the medical device (1) is applied orally, wherein the support component (3) is configured such that each of the at least one push button (4) is displaceable separately relative to the support component (3) between a first position and a second position.

## Description

The invention relates to a medical device for oral procedures, particularly for use in oral intubation and fixation procedures according to claim 1.

Endotracheal intubation is a procedure performed in anesthetised patients in order to secure oxygen supply.

The intubation procedure is performed with a laryngoscope, a device comprising a rigid blade structure that is configured to be inserted in the open mouth of the unconscious patient. The blade structure is designed such that the tongue is put aside towards the left. Furthermore, the intubation with the laryngoscope requires that the blade structure is inserted so deep that the vocal chords can be exposed and a tube can then be safely inserted into the trachea through the vocal chords opening.

During manipulation with the laryngoscope within the oral cavity especially the upper teeth of the patient are endangered, as the upper teeth of the patient often times are used improperly as a fulcrum for the blade structure in order to navigate a distal end of the blade structure towards the opening of the vocal chords. This is particularly the case when inexperienced personal is performing the intubation but also when the air way has a complicated shape.

The pressure and force applied to the teeth by the blade structure can cause tooth damage ranging from splintering up to more severe dental traumata such as a broken tooth or broken teeth.

Likewise, oral insertion, intubation or fixation procedures using different oral medical devices, bear similar risks of tooth damage due to excessive force exposure resulting from direct contact to such devices.

In order to prevent such damages, various solutions have been proposed in the state of the art.

EP 0872209 A1 teaches a blade assembly for a laryngoscope that comprises a resilient protective insert that is configured to deflect a force from the teeth by elastic deformation of the insert.

A similar approach is disclosed in US 20040034281 A1, where a disposable insert for a laryngoscope is arranged at a distal end of the blade structure of the laryngoscope. The insert is made from a soft, deformable material in order to prevent tooth damage.

Following the same concept, US 20070235040 A1 teaches a soft protective pad arranged on the blade structure of a laryngoscope.

However, the deformable materials can come in the line of sight of the surgeon / anesthetist, when being deformed by the teeth of the patient, impairing the intubation procedure.

Moreover, these devices do not limit the applicable pressure on the teeth and are not well suited as quantitative pressure indicators.

A feed-back mechanism on the applied pressure in turn, would alleviate the problems according of the state of the art and help medical personal during intubation procedures to assess the exerted pressure / force on the teeth.

The goal of the invention is therefore to provide an improved solution to the problem of dental trauma caused by medical devices, suited for quantitative pressure feedback.

This object is achieved by the medical device having the features of claim 1.

Advantageous embodiments are described in the subclaims.

According to the invention, a medical device for oral procedures, particularly for use in oral intubation and fixation procedures, comprises at least one protective device for teeth, the protective device comprising the components
- A rigid support component, and
- At least one push button, arranged at the support component, wherein each of the at least one the push button comprises a non-elastic surface that is facing towards, particularly the incisal edges of the upper teeth, when the medical device is applied orally, wherein
the support component is configured such that each of the at least one push button, particularly its rigid surface, is vertically displaceable (that is along the push direction of the push button) separately relative to the support component between a first position and a second position.

The push-button with its rigid surface is particularly shiftable or moveable in a shape-maintaining manner. This protection mechanism stands in stark contrast to the state of the art, where the protective device is deformed by external forces rather than being displaced.

The rigid surface in the context of the present invention relates particularly to an integral part of the of the push-button, wherein said integral part is the designated push-down area of the push button, i.e. said area is configured and designed for receiving a force for pushing down the push button.

The rigid surface area therefore is not necessary the outmost or topmost layer but can be covered with an additional protective layer or coating that might comprise elastic or deformable properties. The displacement between the first and second position of the at least one push button relative to the support component is particularly more than 1 millimetre, more particularly 5 millimetres along a direction towards the support component, when force is applied to the push button. The push button is therefore particularly not displaced parallel but orthogonally towards the support component.

The at least one push button is designed to be pressed down by a tooth. Thus, the push button of the protective device is particularly arranged such on the medical device, that it is directly exposed to the upper or lower teeth of the patient.

A quantitative pressure estimation is possible for example by analysing the position the push button has adopted, namely the first or second position.

Both, the support component and the push button and particularly all other components of the protective device can be made by an injection moulding process.

Thus, the protective device particularly consists at least partially or in full of a plastic that is configured for injection moulding processes.

Alternatively or additionally, the support component and the push button and particularly all other components of the protective device can be made by additive manufacturing, such as 3D-printing processes or selective laser sintering. Thus, the protective device particularly consists at least partially or in full of a plastic or another compound that is configured for additive manufacturing.

According to one embodiment of the invention, the protective device is configured for being repeatedly mounted and dismounted on the medical device. This embodiment allows for convenient replacing of used protective devices such that medical device can be re-used multiple times with an unused protective device.

According to an alternative embodiment of the invention, the protective device is configured for single-use only and is particularly configured for being mounted and dismounted on the medical device only once.

According to another embodiment of the invention, the protective device is configured for multiple uses, wherein such a protective device is particularly configured such that when the at least one push-button is at the second position, it is repositionable towards the first position.

The protective device is of such dimensions that it fits in the mouth without limiting the visualization of the larynx particularly when mounted on the medical device.

According to another embodiment of the invention, the rigid surface comprises a rough surface structure, particularly a relief structure, configured to prevent a lateral movement of the surface with respect to a tooth, such that particularly an enhanced grip of is provided to the medical personal operating with the protective device. Such relief structure can for example comprise lateral, particularly millimetre-sized protrusions that extend essentially orthogonal to an elongated axis of the protective device and/or essentially parallel to the edge of the tooth.

The rough surface structure can comprise an elastic polymer or rubber, particularly an elastic polymer or rubber coating.

According to another embodiment of the invention, the rigid surface has grooves that are designed for reducing slip and increasing grip, preventing anterior-posterior movement of the surface with respect to the tooth. The grooves are particularly arranged crossways to the protective device, i.e. parallel to the cutting edge of the tooth.

According to another embodiment of the invention, the protective device comprises a metal or a metal compound, particularly a medical grade metal or metal compound and/or a polymer, particularly a polymer configured for injection moulding or additive manufacturing as described above.

Such medical grade compounds comprise for example Stainless steel, Titan, coated Aluminum, PEEK (CAS-Nr: 29658-26-2), Polyamide (CAS-Nrs: 25038-54-4, 32131-17-2, 9011-52-3, 26098-55-5, 25035-04-5, and/or 24937-16-4), Polyoxymethylen (POM; CAS-Nr: 9002-81-7), Polyethylen (PE; CAS-Nr: 9002-88-4), Polycarbonates, Polyethylenterephthalat (PET; CAS-Nr: 25038-59-9), Polyvinylidenfluorid (PVDF; CAS-Nr: 24937-79-9), Polysulfon (PSU; CAS-Nr: 25135-51-7).

According to another embodiment of the invention, the surface of the at least one push button comprises an elastic layer arranged on the surface, wherein said elastic layer is particularly deformable and wherein said layer is particularly thinner than 5 millimetre. Said layer can be formed as a coating of the rigid surface.

According to another embodiment of the invention, the rigid surface comprises an elastomeric layer for protecting the push button.

An elastomeric layer or an elastic layer increases the predefined force, as they can absorb a force partially.

According to another embodiment of the invention, the protective device is configured such that the at least one push button is displaced from the first position, only when a force on the surface of the push button exceeds a predefined force, particularly wherein, when the force exceeds said predefined force, the push button snaps, particularly irreversibly in the second position, particularly wherein the protective device is configured to emit an audible sound signal, when the push button is displaced to the second position.

This embodiment provides the medical personal applying the medical device with a feed-back mechanism that allows for quantitative assessment of the applied force on the teeth.

Whenever the push button clicks on the second position, it is unambiguously indicated that the predefined force has been exceeded.

The predefined force is particularly chosen such that it is below the force that can cause dental trauma or dental damage.

The snapping mechanism can be used for generating an audible sound signal. The audible sound signal is an additional feed-back mechanism that is configured to indicate the medical personal that the force applied to the teeth has been exceeded the predefined force.

This is particularly useful as medical personal might be concentrating on the oral procedure, e.g. the intubation rather than watching the push button being displaced. With the additional audible sound signal, the medical personal receives a feedback, indicating that the predefined force has been exceeded.

Such a binary response mechanism can be for example realized by using a clicker or other suitable means.

It is possible to design the displacement of the push button in an irreversible way such that once the push button has been displaced, the push button remains in the second position even though the force applied to the push button might have ceased or is even inverted.

According to another embodiment of the invention, the support component and each of the at least one the push button comprise complementary means for a snap-fit mechanism, wherein, when the force on the surface of the push button exceeds the predefined force, the push button snaps by means of the snap-fit mechanism in the second position, particularly wherein an audible sound signal is produced by the snap-fit means, when the push button snaps in the second position.

An embodiment comprising a snap-fit mechanism can be realized very economically for example in an injection moulding process or an additive manufacturing process as detailed above, allowing the protective device being manufactured for example from plastic only.

The snap-fit mechanism has the advantage of being designable to engage at a predefined force and for simultaneously providing an audible sound signal to the medical personal when it is engaging.

By designing the snap-fit means accordingly, basically any force condition can be met at which the snap-fit mechanism snaps in the second position.

According to another embodiment of the invention, the support component comprises a snap-fit means in form of a first snap-fit nut and a second snap-fit nut and the push button comprises a snap-fit cantilever, wherein in the first position, the push button is arranged such at the support component that the snap-fit cantilever is engaged in the first snap-fit nut, and when the force on the surface of the push button exceeds said predefined force, the push button is displaced towards the second position, particularly as the snap-fit cantilever is swept out of the first snap-fit nut by the external force, wherein the push button snaps in the second position, as the cantilever engages with the second snap-fit nut.

According to another embodiment of the invention, the support component comprises opposing wall portions and a bottom portion, wherein the wall portions and the bottom portion form a recess, wherein the push button is arranged at an upper end of the opposing wall portions, wherein particularly the snap-fit means of the support component are arranged at the wall portions facing outwards the recess.

The wall portions particularly extend along the two sides of the protective device that face the corners of the mouth, when the protective device is insert in the mouth of a patient. The recess of the protective device is particularly formed as a channel that is open towards an upper side or upside, where the push button is located.

According to this embodiment the push button is therefore arranged opposite of the bottom portion.

This embodiment allows for the arrangement of various components in the recess, and /or that the recess can be designed as a guiding structure for inserting the at least one push button. The guiding structure can also serve as a resonance cavity for the signal sound.

According to another embodiment of the invention, the recess is tapered towards the upper side.

According to another embodiment of the invention, the push button comprises an anchor portion that reaches in the recess of the support component.

Said anchor portion is particularly designed complementary to the recess such that the recess is at least partially filled with the anchor portion of the push button. The push button comprising the anchor portion is displaceable only towards the bottom portion of the recess.

According to another embodiment of the invention, the anchor portion is at least partially hollow, particularly forming a resonance cavity that is configured to resonate sound originating from a displacement of the push button from the first to the second position, particularly the signal-sound of the snapping snap-fit mechanism.

According to this embodiment, the recess and the at least partially hollow anchor portion is configured to resonate a sound stemming from a mechanical displacement of the push button.

As hollow bodies are better suited to resonate any sound, this embodiment realizes a sound indicator as feed-back mechanism for the medical personal.

According to another embodiment of the invention, particularly for each push button a particularly preloaded spring or clicker is arranged in the recess of the support component, wherein the clicker is configured to click, i.e. emit an audible sound signal, when the push button is displaced from the first to the second position, particularly wherein the spring is arranged with a first end at the bottom portion of the support component and with a second end at the anchor portion, particularly in the resonance cavity.

A clicker is an alternative or additional embodiment with regard to embodiments based on a snap-in mechanism that allows for audio feed-back.

The audio-feedback signal can also be provided or generated by an electronic or electrically controlled acoustic means that is for example triggered by the spring or the clicker.

A spring loaded push button is suited for a continuous visual feed-back of the applied force, as the spring can be chosen such it responds particularly linearly to an external force.

According to another embodiment of the invention, the protective device comprises a plurality of push buttons, particularly two to eight push buttons, more particularly 5, 6, 7 or 8 push buttons that are arranged in a row, adjacent and adjoining to each other on the support component.

But also a protective device with more push buttons, such as for example 6 to 10 push buttons, are suitable, depending on the size, particularly the length, of the protective device.

This embodiment provides a high degree of flexibility for varying oral anatomies of patient. Independent of the anatomy and/or independent of the depth the medical device is inserted into the mouth of the patient, the embodiment always reliably provides a push button arranged opposite the tooth row. This embodiment furthermore allows that the push buttons can be designed such that they respond ideally to an external force that might act in a tilted manner on the push button. Push buttons with a too large rigid area might not respond reliable, when for example the exerted force is not attacking in the centre of the surface but on one side of the surface, leading to an asymmetric force profile on the protective device that might lead to a tilt rather than to a push down of the push button.

By arranging a plurality of push buttons in a row, each push button can serve as a feed-back indicator for the medical personal. As each push button for example comprises some mechanism to provide an audible signal sound, when displaced in the second positon, the medical personal does not need to focus on the exact location of the protective device, when for example the teeth are accidently or improperly used as a fulcrum for the medical device.

According to this embodiment of invention, particularly the responsive area of the protective device is increased, while maintaining the reliability of response of the protective device.

According to another embodiment of the invention, the support component extends along an elongated axis extending along the row of push buttons, wherein the support component comprises a guiding structure extending along the elongated axis for providing a support for the push buttons on the support component and wherein the guiding structure particularly comprises the snap-fit means of the support component, wherein the guiding structure is configured to receive the push buttons from a proximal end of the guiding structure arranged at an proximal end of the support component, wherein the push buttons are inserted in said guiding structure, particularly wherein the anchor portion comprises anchor means that are configured to engage in the guiding structure of the support component.

The anchoring means particularly fix the push buttons in the recess so that they do not fall off the support component.

According to this embodiment the protective device can be assembled by sliding the push buttons in the guiding structure, where the anchoring means protrude in such a manner in the recess that the push buttons cannot fall of the support component.

According to another embodiment of the invention, the support component comprises a lock at the proximal end of the support component, wherein the lock exerts a transversal force on the adjacently arranged push buttons such that the push buttons are fixed adjacently in a row in the guiding structure.

The lock can be a spring or a spring loaded device that particularly fixes the position of the push buttons in the guiding means laterally. The lock can also be made of plastic that can be injection moulded.

According to another embodiment of the invention, the protective device and particularly all its components consists of a plastic adapted for injection moulding. Injection moulding is a suitable mass-production process that allows to produce the protective device in a very cost efficient manner. Such a protective device is particularly less expensive than rubber materials or other deformable, soft materials used in state of the art protective devices.

According to another embodiment of the invention, the protective device is configured to generate an acoustic signal-sound for indicating a displacement of the push-button, wherein said signal-sound is produced when or after the push-button is displaced from the first position to the second position, wherein said audio-signal is particularly produced by either mechanical means or by an electronically- or electrically-controlled acoustic device, such as a loudspeaker.

The signal-sound is therefore an additional protective feature of the medical device for preventing damage to the teeth.

The signal.-sound can be produced by purely mechanical means, such as for example a snap-fit mechanism, when it snaps from a first position to a second position.

Alternatively a clicker is configured to produce such an audible sound when it clicks.

Also electronical or electrical devices configured for producing a sound, such as a loudspeaker with a corresponding activation means and energy source can be used for producing such a signal-sound.

The activation of the electric or electronic signal-sound means can for example be realized by an electric contact established between for example the anchor portion and the support component that is put in contact, when the push-button is displaced to the second position. But also other activation mechanisms are known to the person skilled in the art. Also there exists a variety of suitable electric or electronic sound-signal devices for the medical device according to the invention.

According to another embodiment of the invention, the predefined force is between 5 N to 200 N, particularly between 10 N and 100 N, more particularly between 15 N and 30 N. The predefined force is particularly adjusted such that it is below the force that might induce trauma or injury to the teeth.

Particularly a predefined force between 15 N and 30 N prevents tooth damage or trauma and allows for convenient handling of the protective device.

In one embodiment, the predefined force is between 20 N and 25 N, when the push button is moved for the first time from the first to the second position and wherein the predefined force is particularly lower, when the push button is pressed down repeatedly, wherein particularly the predefined force becomes lower each time the push button is pressed.

As the protective device is particularly designed as a disposable, i.e. for single-use, the reduction in predefined force for repeated uses is insignificant. A protective device being sensitive to this force is suitable for protecting the teeth of patient, particularly when the teeth are used as a fulcrum for the medical device.

According to another embodiment of the invention, the medical device comprises a plurality of protective devices.

According to another embodiment of the invention, the medical device is a blade assembly for a laryngoscope, the blade assembly comprising an elongated blade structure for oral or nasal intubation procedures, wherein the at least one protective device is arranged such at the blade structure that when the blade assembly is orally inserted in a patient, the protective devices faces the upper teeth, particularly the incisors, with its at least one push button.

Such a blade assembly solves the problem according to the invention in an advantageous manner for laryngoscopes. In comparison to blade assemblies known form the state of the art, said blade assembly is configured for receiving a protective device at the appropriate position.

The appropriate position of the protective device is particularly at a proximal end of the blade structure.

According to another embodiment of the invention, the elongated blade structure has a proximal and a distal end, wherein the at least one protective device is arranged at the proximal end of the blade structure, wherein blade structure particularly comprises a retention channel that is designed to receive a retention ridge of the at least one protective device, and wherein the retention channel is adapted provide guidance and retention for the at least one protective device.

This embodiment allows the re-use of the blade structure by simply replacing a used protective device via the retention channel.

According to another embodiment the medical device is a laryngoscope comprising the blade assembly.

The laryngoscope according to the invention would comprise a feed-back mechanism that at the same time limits the force applied to the teeth of the patient. The view on the larynx entry would not be obstructed and the replaceable protective device would allow a comparably hygienic solution for intubation procedures as for example no change of gloves would be necessary.

According to another embodiment of the invention, the laryngoscope comprises a fibre optic and a light source connected to the fibre optic, wherein the fibre optic is arranged such on the laryngoscope that the throat can be illuminated.

Illumination helps to guide the laryngoscope more precisely during intubation procedures.

Further features and advantages of the invention shall be described by means of a detailed figure description, wherein features disclosed in the figure section can also be used in combination with the claimed subject matter.

It is shown in
Fig. 1 a perspective view of a protective device with five push buttons;
Fig. 2 a cross-section and a perspective view of a protective device with a snap-fit mechanism facing outwards the protective device and an enlarged hollow anchor portion;
Fig. 3 a cross-section of a protective device with a spring and a snap-fit mechanism facing inwards the protective device;
Fig. 4 a cross-section of a different protective device with a spring and a snap-fit mechanism facing inwards the protective device;
Fig. 5 a cross-sectional view of a protective device with a hollow anchor portion, a spring and a snap-fit mechanism facing outwards the protective device;
Fig. 6 a cross-sectional view of a protective device with an enlarged hollow anchor portion, a spring and a snap-fit mechanism facing outwards the protective device;
Fig. 7 a cross-sectional view and a perspective view of a protective device with a massive and a snap-fit mechanism facing outwards the protective device;
Fig. 8 a perspective view of a blade assembly for a laryngoscope comprising a protective device; and
Fig. 9A to Fig. 9C measured maximum forces applied to the protective device corresponding to the predefined force of the protective device.

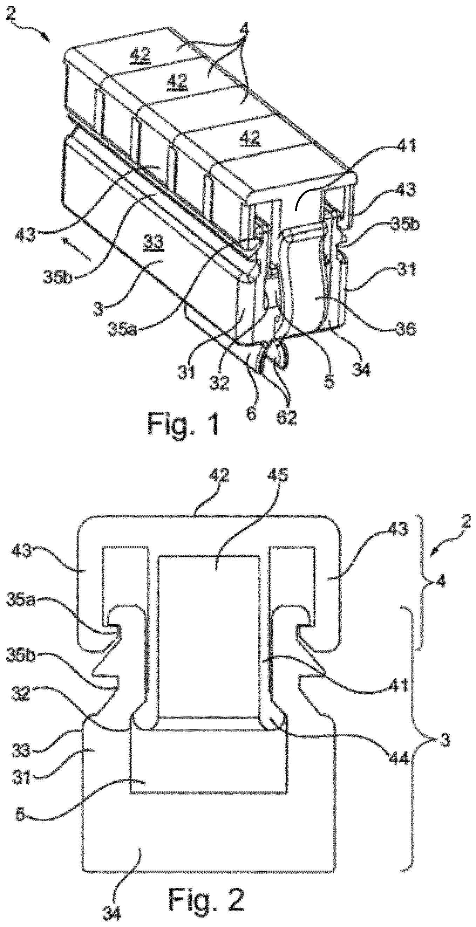

Fig. 1 shows a perspective view of a protective device 2 and Fig. 2 shows a corresponding cross-section through the protective device 2. The protective device 2 comprises a support component 3 on which five push buttons 4 are arranged.

The support component 3 has an elongated shape and comprises two wall portions 31 that extend along the elongated axis (indicated by the arrow) of the support component 3. The walls 31 each have an outer side 33 that faces outwards the support component 3 and an inner side 32 facing a recess 5 inside the support component 3.

The recess 5 of the support component 3 is limited on its bottom by a bottom portion 34 of the support component 3.

The recess 5 of the support component 3 is tapered towards an upper side of the recess 5.

On the outer side of the bottom portion 34 a retention ridge 6 is arranged such on the support component 3 that the support component 3 and thus the protective device 2 can be stably slid into a medical device comprising a complementary retention channel 61 for the retention ridge 6.

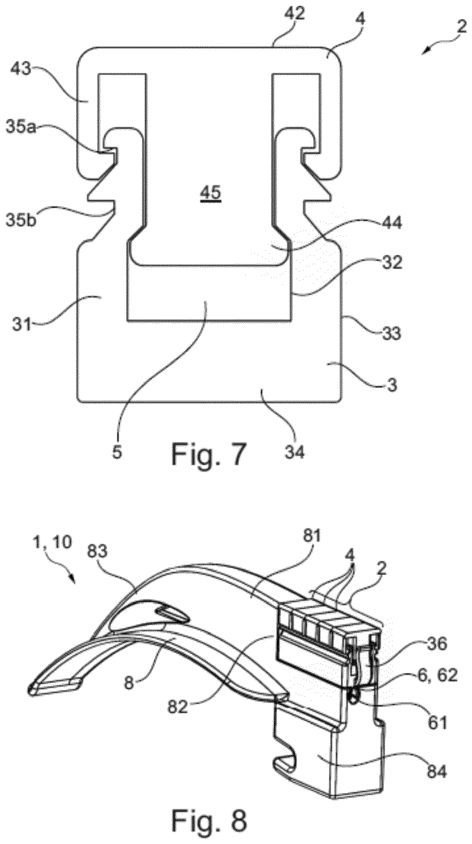

The retention ridge 6 is formed such that it exerts a clamping force by means of two spring elements 62 on the retention channel 61 (shown in Fig. 8) such that the support component 3 is retained in the retention channel 61.

An upper portion of the support component 3 is open.

Furthermore, each wall portion 31 comprises on its upper portion of its outer sides 33 two snap-fit nuts 35a, 35b that extend along the elongated axis of the support component 3, wherein a first snap-fit nut 35a is extending above a second snap fit nut 35b.

The five push buttons 4 are arranged adjoining and adjacent to each other in a row along the elongated axis of the support component 3. Each push button 4 comprises an anchor portion 41 that extends into the recess 5 of the support component 3, a rigid surface 42, and two snap-fit cantilevers 43 embracing the upper portions of the wall portions 31 of the support component 3. The snap-fit cantilevers 43 of the push button 4 are engaged in the first snap-fit nut 35a, when the push button 4 is in a first position. The snap-fit cantilevers 43 act as springs that hold the push button 4 in the first position. When a force is applied to the up-facing rigid surface 42 of any of the five push button s 4 that exceeds the predefined force, for example 30 N or 20 N, the respective push button 4 will snap into a second position that corresponds to the snap-fit cantilevers 43 of the push button 4 being engaged in the second snap-fit nut 35b.

Thus, the two snap-fit cantilevers 43, to be more precise the spring constants of the two snap-fit cantilevers 43, are adjusted such that the snap-fit cantilevers 43 disengage from the first snap-fit nut 35a, when the applied force to the rigid surface 42 of the push button 4 is exceeding the predefined force, rendering the push button 4 displaceable towards the second position of the push button 4, wherein the second position is defined by the engaging of the cantilevers 43 in to the second snap-fit nut 35b.

The up-facing rigid surface 42 is configured to receive the (counter-) force of the upper teeth of a patient when the protective device 1 is pushed against the teeth.

The anchor portion 41 of the push button 4 is partially shaped complementary to the tapered recess 5, wherein particularly at a lower portion of the anchor portion 41, the push button 4 comprises protrusions 44 that are configured to inhibit a potential displacement of the push button 4 outwards the recess 5.

The tapered recess 5 in combination with the snap-fit nuts 35a, 35b provides a guiding structure extending along the elongated axis of the support component 3 for the push buttons 4.

When the protective device 2 is being assembled said guiding structure helps to slide push button s 4 into the support component 3 along the elongated axis.

The anchor portion 41 is at least partially hollow 45 such that a sound produced by the snap-fit means 43 of the push button 4 and the support component 3 resonates in said hollow anchor portion 45. This hollow portion 45 particularly amplifies a snapping sound such that a comparably loud sound signal is produced, when the cantilevers 43 are snapping in the second snap-fit nut 35b. The sound signal can serve as an indicator or feedback for medical personnel working with a medical device 1 comprising the protective device 2, indicating that the force applied to the teeth has exceeded the predefined force.

Therefore, the protective device 2 indicates not only visually that a predefined force has been exceeded but also in an audible manner. Medical personnel can therefore focus on the medical procedure without having an eye on the protective device 2.

In order to keep the five push buttons 4 adjoining in a row next to each other, the elongated support component 3 comprises on a proximal end a lock 36 that is also configured to exert a longitudinal force along the elongated axis on the anchor means 41. Set force keeps the five push button s 4 in a row and prevents a loss from the proximal end.

The protective device 2 is made of plastic or a polymer and can be produced by means of injection moulding or by additive manufacturing, such as selective laser sintering, wherein the latter allows other compounds than polymers to be used.

In the following, identical reference signs refer to the same feature of the specific embodiment as the features outlined in Fig. 1 and Fig. 2, even if not explicitly stated.

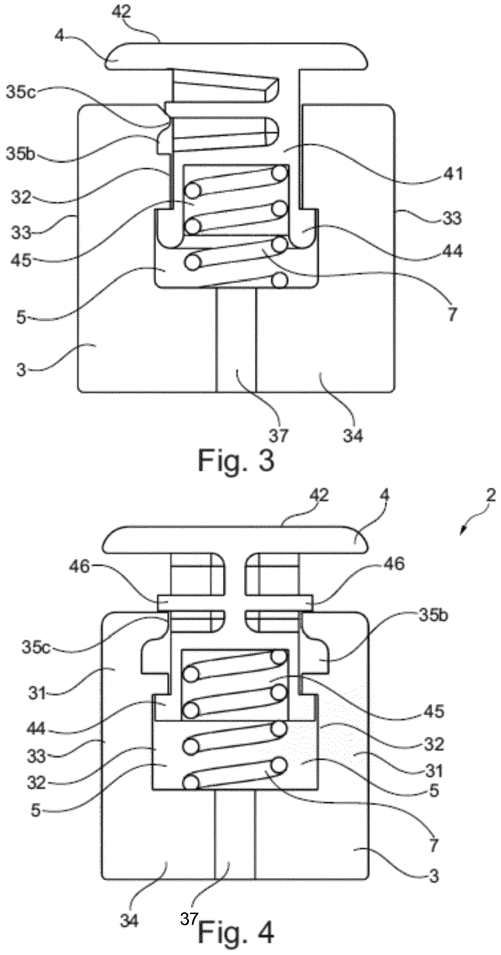

Fig. 3 shows another example of a protective device. Here, the snap fit mechanism is located the inner side 32 of the wall portion. One snap fit nut 35b is located below a protrusion 35c that poses an obstacle for the push button 4 such that the push button 4 remains in the first position until an external force is high enough to overcome the obstacle and the protrusion is pushed aside by the push button 4 such that the push button 4 switches to the second position. The push button 4 comprises a hollow anchor portion 45 that extends inside the recess 5 of the support component 3. At the lower end of the anchor portion 41, the anchor portion 41 comprises protrusions 44 that are configured to keep the push button connected to the support component 3. A loaded spring 7 extends from the bottom portion 34 of the support component 3 into the hollow portion 45 of the anchor portion 41. On the upper end of the spring 7 the spring touches the ceiling portion of the hollow portion of the anchor portion 41, such that a predefined force is exerted on the push button 4 pointing upwards and potentially against a force directed against the spring force. The spring 7 can be used for a precise control of the force needed to push down the push button 4. Thus, the embodiment in Fig. 3 has two mechanisms to counter a force pushing the push button 4 downwards in the second position; the snap fit mechanism 35b and 35 c and the spring 7. The support component 3 comprises on its bottom portion a channel 37 that is configured as a repositioning access for a pin or a specific tool for repositioning a push button that is snapped in the second position. The pin or tool can be inserted through the channel 37. With the inserted pin or tool a push-button can be released form the second position and repositioned to the first position allowing a re-use of the protective device. This is particularly useful for multi-use designs of protective devices.

Fig. 4 shows a cross section of a variation of the embodiment shown in Fig. 3. In Fig. 4 the support component 3 is essentially symmetrical. The support component 3 comprises two snap fit mechanisms on each wall portion 31 that grant a symmetric response characteristic of the protective device 2. Similar to the device 2 shown in Fig. 3 the protective device 2 comprises a particularly preloaded spring 7 arranged in the recess 5 of the support component 3. The protrusions 44 at the lower end of the anchor portion 41 are of different shape but have the same function.

The snap-fit mechanism comprises a snap-fit nut 35b, a protrusion 35c on the inner side 33 of the wall portion 31 and a protruding element 46 arranged at the push button 4. By applying a force pointing inwards the recess 5, the resistance of the snap-fit mechanism can be overcome and the protruding element 46 snaps into the snap-fit nut 35b.

Analogously to the protective device 2 shown in Fig. 3, also the device 2 shown in Fig. 4 comprises a channel 37 arranged at the bottom portion 34 of the support component 3.

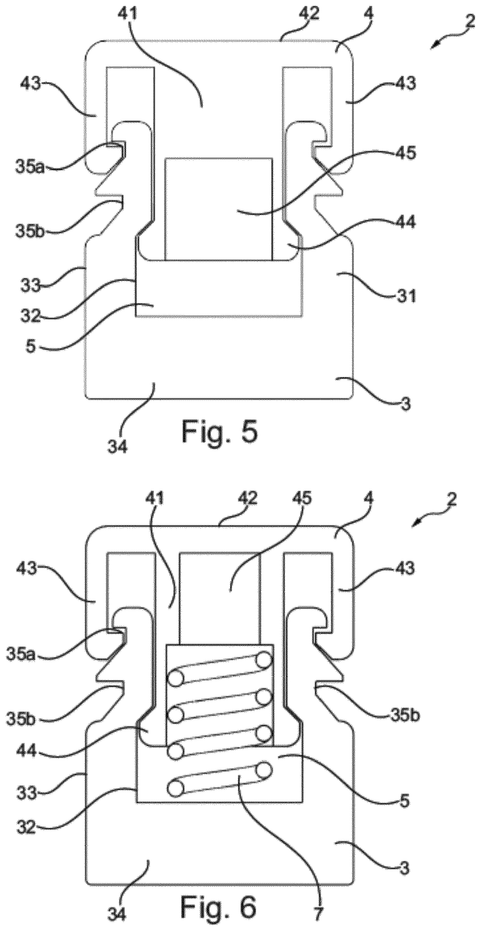

In Fig. 5 an essentially identical protective device 2 is shown as the protective device 2 shown in Fig. 2. Here however, the hollow portion 45 of the anchor portion 41 is smaller than the hollow portion 45 shown in Fig. 2.

This leads to a more stable push button 4 design, but at the same time sound amplification and resonating characteristics of the protective device 2 of the snap-fit mechanism are reduced.

In Fig. 6 a spring 7 is arranged in the recess 5 of the support component 3 and in the hollow portion 45 of the anchor portion 41. The spring 7 is arranged such that it counters a force pointing inwards the recess 5 of protective device 2, pushing the spring 7 together. An upper portion of the hollow portion 45 of the anchor portion 41 is left empty such that the amplification and resonating behaviour of the push button 4 is maintained.

In Fig. 7 a protective device 2 essentially identical to the protective device 2 in Fig. 2 is shown, with the difference that the anchor portion 41 is completely massive and has no hollow portion. This design provides a more stable push button design but at the same time sound made by the snap-fit mechanism is not amplified nor does is resonate as well.

In Fig. 8 a blade assembly 8 for a laryngoscope is depicted. The blade assembly 8 comprises an elongated blade structure 81. The blade assembly shown in Fig. 8 is similar to blade assemblies known from the state of the art with the specific difference that the depicted blade assembly 8 is designed such that a protective device 2 according to the invention can be attached to said blade assembly 8 at an proximal end 82 such that it can serve as an indicator of applied pressure on the teeth of a patient once it is used. The push buttons 4 of the protective device 2 complete with an upper portion of the elongated late structure 81. The blade assembly 8 comprises a retention channel 61 that is configured to receive a retention ridge 6 of the protective device 2. On its distal end there's also a handle 84 that can be used for attaching the blade assembly 8 to a laryngoscope.

The blade assembly 8 is inserted to the mouth of a patient with its distal proximal end 83.

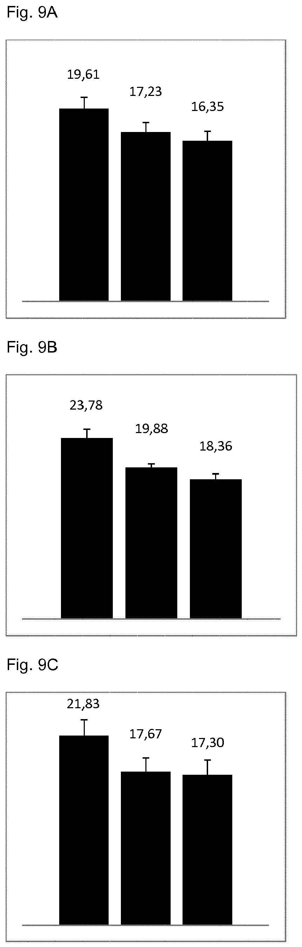

Fig. 9A, Fig. 9B and Fig. 9C show the predefined force for three different embodiments of the invention. In Fig. 9A the push button of the protective device has a rigid unstructured surface. In Fig. 9B the rigid surface is covered with an elastomeric layer and in Fig. 9C the rigid surface comprises grooves.

Each push button of the respective device was pushed down three times and the force was measured. The average value (n=3) of the measured force for three devices of the respective embodiment is plotted as a bar and represents the predefined force. The left bar in Figure corresponds to the first attempt, the middle bar corresponds to the second attempt and the right bar corresponds to the third attempt. Each time the push button is pushed down the measured force is reduced by some extent. The measured force is written on top of each bar.

## Claims

1. A medical device (1) for oral procedures, particularly for use in oral intubation and fixation procedures, comprising at least one protective device (2) for teeth, the protective device (2) comprising the components
- A rigid support component (3), and
- At least one push button (4), arranged at the support component (3),
wherein each of the at least one the push button (4) comprises a rigid surface (42) that is facing towards the teeth, when the medical device (1) is applied orally
**characterized in that**
the support component (3) is configured such that each of the at least one push button (4) is displaceable separately relative to the support component (3) between a first position and a second position.

2. Medical device according to claim 1, wherein the protective device (2) is configured such that the at least one push button (4) is displaced from the first position only when a force on the surface (42) of the push button (4) exceeds a predefined force, particularly wherein, when the force exceeds said predefined force, the push button (4) snaps, particularly irreversibly in the second position, particularly wherein the protective device (2) is configured to emit an audible signal-sound, when the push button (4) is displaced to the second position.

3. Medical device according to claim 2, wherein the support component (3) and each of the at least one the push button (4) comprise complementary means for a snap-fit mechanism, wherein, when the force on the surface (42) of the push button (4) exceeds the predefined force, the push button (4) snaps by means of the snap-fit mechanism in the second position, particularly wherein an audible signal-sound is produced by the snap-fit means, when the push button (4) snaps in the second position.

4. Medical device according to claim 3, wherein the support component (3) comprises a snap-fit means in form of a first snap-fit nut (35a) and a second snap-fit nut (35b) and the push button (4) comprises a snap-fit cantilever (43), wherein in the first position, the push button (4) is arranged such at the support component (3) that the snap-fit cantilever (43) is engaged in the first snap-fit nut (35a), and when the force on the surface (42) of the push button (4) exceeds said predefined force, the push button (4) is displaced towards the second position, where the push button (4) is arranged such at the support component (3) that the snap-fit cantilever (43) is engaged in the second snap-fit nut (35b).

5. Medical device according to one of the preceding claims, wherein the support component (3) comprises opposing wall portions (31) and a bottom portion (34), wherein the wall portions (31) and the bottom portion (34) form a recess (5), wherein the push button (4) is arranged at an upper end of the opposing wall portions (31), wherein particularly the snap-fit means of the support component (3) are arranged at the wall portions (31) facing outwards the recess (5).

6. Medical device according to claim 5, wherein the push button (4) comprises an anchor portion (41) that is reaches in the recess (5) of the support component (3).

7. Medical device according to claim 6, wherein the anchor portion (41) is at least partially hollow, particularly forming a resonance cavity (45) that is configured to resonate sound originating from a displacement of the push button (4) from the first to the second position, particularly the signal-sound of the snapping snap-fit mechanism.

8. Medical device according to one of the preceding claims, wherein a spring (7) or a clicker is arranged in the recess (5) of the support component (3), wherein the clicker is configured to click, when the push button (4) is displaced from the first to the second position, particularly wherein the spring (7) is arranged with a first end at the bottom portion (34) of the support component (3) and with a second end at the anchor portion (41), particularly in the resonance cavity (45).

9. Medical device according to one of the preceding claims, wherein the at least one protective device (2) comprises a plurality of push buttons (4), particularly two to eight push buttons (4), more particularly 5, 6, 7 or 8 push buttons (4), that are arranged in a row, adjacent to each other on the support component (3).

10. Medical device according to claim 9, wherein the support component (3) extends along an elongated axis extending along the row of push buttons (4), wherein the support component (3) comprises a guiding structure extending along the elongated axis for providing a support for the push buttons (4) on the support component (3) and wherein the guiding structure particularly comprises the snap-fit means of the support component (3), wherein the guiding structure is configured to receive the push buttons (4) from a proximal end of the guiding structure at an proximal end of the support component (3), wherein the push buttons (4) are inserted in said guiding structure, particularly wherein the anchor portion (41) comprises anchor means (44) that are configured to be guided in the guiding structure of the support component (3).

11. Medical device according to claim 10, wherein the support component (3) comprises a lock (36) at the proximal end of the support component (3), wherein the lock (36) exerts a transversal force on the adjacently arranged push buttons (4) such that the push buttons (4) are fixed adjacently in a row in the guiding structure.

12. Medical device according to one of the preceding claims, wherein the protective device (2) is configured to generate an audible signal-sound for indicating a displacement of the at least one push-button (4), wherein said signal-sound is produced when or after the push button (4) is displaced from the first position to the second position, wherein said signal-sound is particularly produced by either mechanical means or by an electronically- or electrically-controlled acoustic device, such as a loudspeaker.

13. Medical device according to one of the preceding claims, wherein the medical device (1) is a blade assembly (8) for a laryngoscope (10), the blade assembly (8) comprising an elongated blade structure (81) for oral intubation procedures, wherein the at least one protective device (2) is arranged such at the blade structure (81) that when the blade assembly (8) is orally inserted in a patient, the protective devices (2) faces the upper teeth with its at least one push button (4).

14. Medical device according to claim 13, wherein the elongated blade structure (81) has a proximal (82) and a distal end (83), wherein the at least one protective device (2) is arranged at the proximal end (82) of the blade structure (81), wherein blade structure (81) particularly comprises a retention channel (61) that is designed to receive a retention ridge (6) of the at least one protective device (2), and wherein the retention channel (61) is adapted provide guidance and retention for the at least one protective device (2).

15. Medical device according to one of the claims 14 or 15, wherein the medical device (1) is a laryngoscope (10) comprising the blade assembly (8).
